# EUROPEAN PATENT APPLICATION

(11) **EP 3 875 457 A1**
(43) Date of publication of application: **08.09.2021**
(21) Application number: 19874995.4
(22) Date of filing: 18.07.2019
(51) Int. Cl.: C07D 471/04, A61K 31/4439, A61P 7/02

(54) **METHOD FOR PREPARING APIXABAN**

(30) Priority: 24.10.2018 KR 20180127214
(71) Applicant: Hana Pharm. Co. Ltd., Hwaseong-si, Gyeonggi-do 18608 (KR)
(72) Inventor: BAEK, Ki Seon, 16604 Suwon-si, Gyeonggi-do (KR); YU, Ji Hye, 16387 Suwon-si, Gyeonggi-do (KR); KANG, Sung Gu, 15563 Ansan-si, Gyeonggi-do (KR)
(74) Representative: V.O.
(86) International application number: PCT/KR2019/008892
(87) International publication number: WO 2020/085616

(57) **Abstract**

The present disclosure relates to a novel method for preparing apixaban. The method of the present disclosure is economical since no expensive reagents or solvents are used. In addition, the method is applicable to mass production on an industrial scale because it requires no special conditions or complicated processes and no hazardous reagents or solvents are used. Furthermore, because no toxic solvent is used in the final step of apixaban synthesis and in a purification process, high-purity apixaban can be prepared stably at high yield without the concern of residual solvents.

## Description

### [Technical Field]

The present disclosure relates to a novel method for preparing apixaban.

### [Background Art]

Apixaban is a known compound having a structure of Chemical Formula 1.

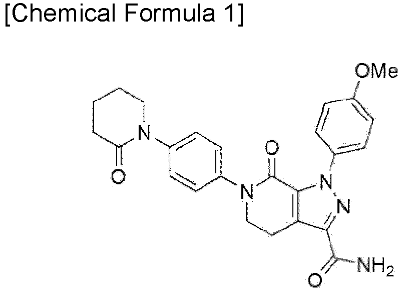

The chemical name of apixaban is 4,5,6,7-tetrahydro-1-(4-methoxyphenyl)-7-oxo-6-[4-(2-oxo-1-piperidinyl)phenyl]-1H-pyrazolo[3,4-c]pyridine-3-carboxamide (CAS name) or 1-(4-methoxyphenyl)-7-oxo-6-[4-(2-oxo-1-piperidinyl)phenyl]-4,5,6,7-tetrahydro-1H-pyrazolo[3,4-c]pyridine-3-carboxamide (IUPAC name).

Apixaban is an anticoagulant, anti-thrombotic factor Xa inhibitor developed by Bristol-Myers Squibb. It has been developed as a medication for oral administration in various indications requiring the use of anti-thrombotic agents. Apixaban is sold under the brand name Eliquis (registered trademark).

Until now, several synthesis methods of apixaban have been known. Apixaban was first disclosed in European Patent Registration No. 1427415 by Bristol-Myers Squibb, wherein apixaban and a method for preparing the same are described. The method described in the patent exhibits very low yield because it involves a number of processes for preparing apixaban. In addition, the method is not suitable for industrial scale because it requires purification by column chromatography. Furthermore, this method is disadvantageous in terms of long reaction time and use of toxic solvents (solvents whose use in pharmaceuticals in has to be avoided).

Meanwhile, WO2003-049681 (patent document 2) by Bristol-Myers Squibb discloses synthesis of apixaban through two pathways. First, Scheme 1 disclosed in the literature is a method of preparing apixaban by converting a carboxylic acid to an amide, wherein 1-(4-methoxyphenyl)-7-oxo-6-(4-2-oxopiperidin-1-yl)phenyl)-4,5,6,7-tetrahydro-1 H-pyrazolo[3,4-c]pyridine-3-carboxylic anhydride is formed and reacted. This reaction process is not commercially viable because expensive iodide is used. In addition, there are disadvantages that reaction conditions are complicated. For example, the reaction should be conducted at low temperature and the process is sensitive to moisture.

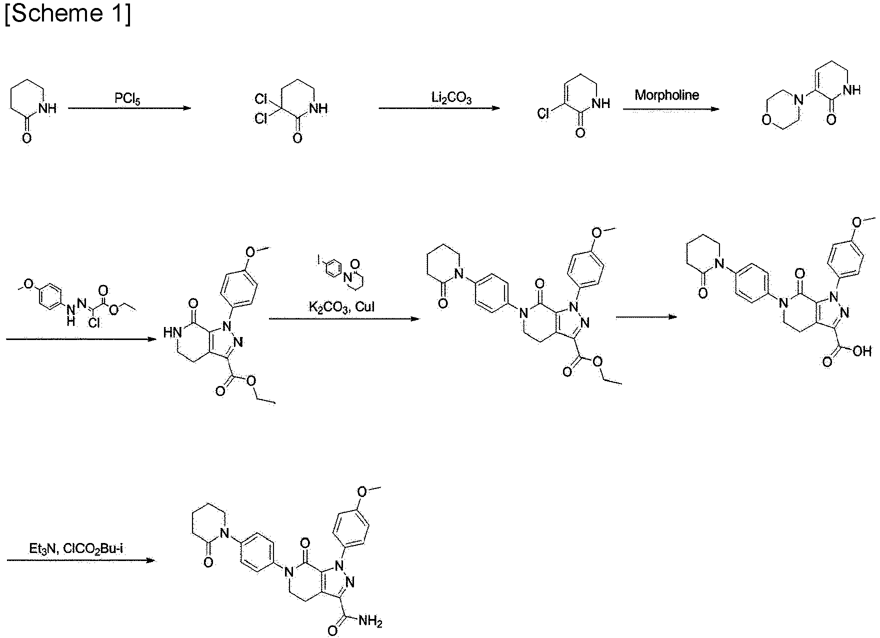

In Scheme 2, which is also disclosed in the literature, apixaban is prepared by converting an ester to an amide. This method is not commercially feasible because the process is costly and sodium methoxide and formaldehyde, which may threaten the safety of operators, are used. In addition, the use of bromotris(triphenylphosphine)copper(I) disclosed in the literature has the problem that the heavy metal copper may remain in the final product and toxic gas may be generated.

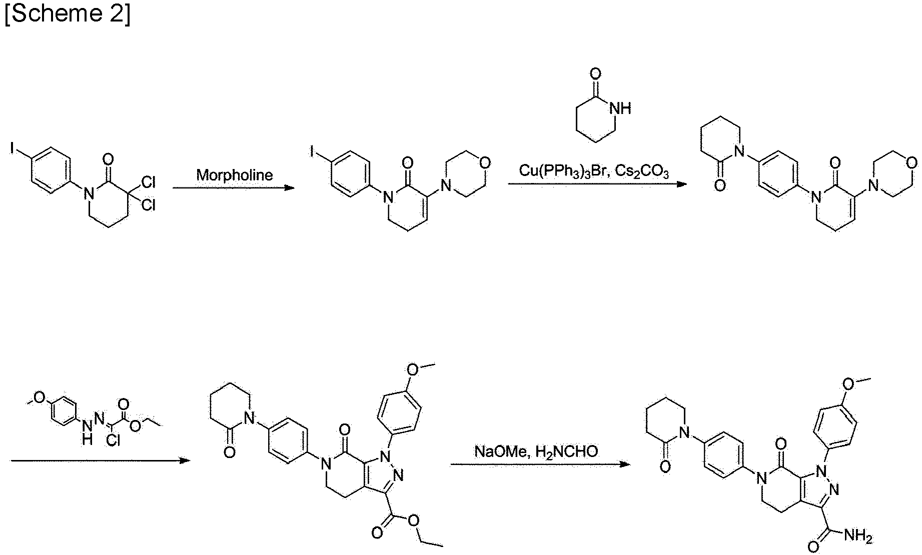

Scheme 3 (non-patent document 1) is a method of preparing apixaban by converting an ester to an amide, which requires, in particular, high-temperature and high-pressure conditions. This method is not commercially viable because the high-temperature and high-pressure conditions requires special production facilities. In addition, when apixaban is synthesized from ethyl-1-(4-methoxyphenyl)-7-oxo-6-(4-2-oxopiperidin-1-yl)phenyl-4,5,6,7-tetrahy dro-1H-pyrazolo[3,4-c]pyridine-3-carboxylate as described in the literature, purification is difficult because acid impurity is produced. Furthermore, the use of ethylene glycol described in the literature is not suitable for a mass production process because it is difficult to completely removing the ethylene glycol which has a high boiling point.

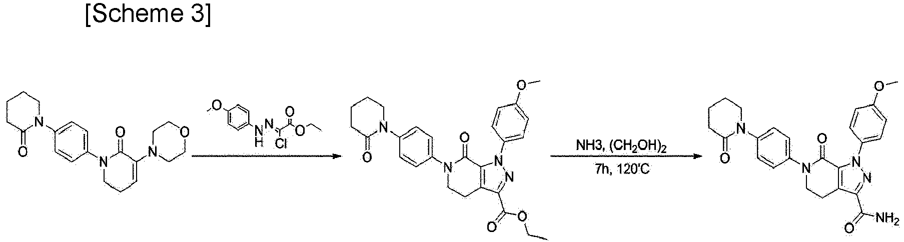

Scheme 4 (patent document 6) is a method of synthesizing apixaban by converting a carboxylic acid to an amide using cyanuric chloride. Although this method may have good reactivity, byproducts may cause toxicity and pollution problems and mass production is not easy because the method is sensitive to moisture.

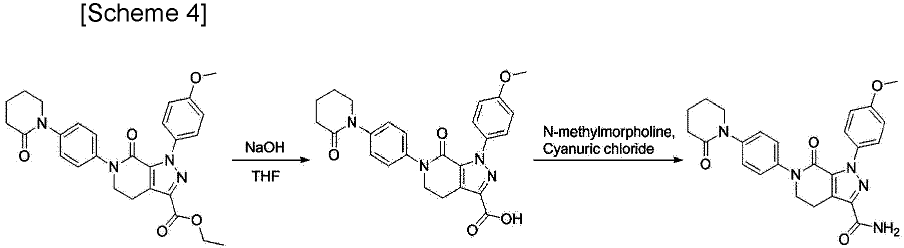

Scheme 5 (patent document 7) is a method of synthesizing apixaban using 1,1'-carbodiimidazole. Although apixaban is synthesized by converting a carboxylic acid to an amide in this method, it is not commercially viable because 1,1'-carbodiimidazole is expensive.

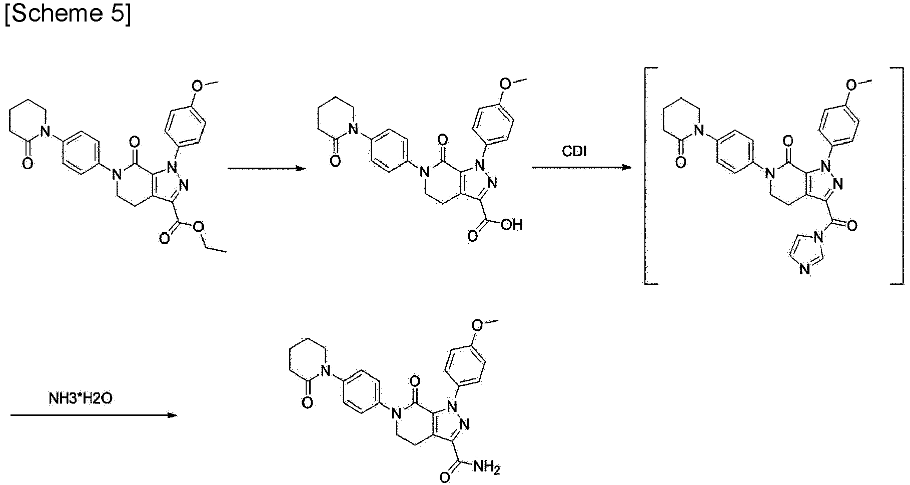

Under this background, the inventors of the present disclosure have researched to develop a method capable of preparing high-purity apixaban economically and effectively at high yield without requiring expensive reagents or special production facilities. They have found out that preparation of apixaban from 2-benzothiazolyl-1-(4-methoxyphenyl)-7-oxo-6-(4-2-oxopiperidin-1-yl)phenyl)-4, 5,6,7-tetrahydro-1H-pyrazolo[3,4-c]pyridine-3-carboxylate allows easy removal of byproducts and also allows mass production by solving the problems of the existing methods, and have completed a method for preparing apixaban using the same.

### [References of Related Art]

### [Patent Documents]

(Patent document 001) WO2003026652 A.
(Patent document 002) WO2003049681 A.
(Patent document 003) WO2007001385 A.
(Patent document 004) WO2010030983 A.
(Patent document 005) WO2012168364 A.
(Patent document 006) EP3228619 A.
(Patent document 007) CN104045637 B.
(Patent document 008) CN104844593 A.

### [Non-patent Documents]

(Non-patent document 001) J. Med. Chem. 2007, 50, 5339-5356.

### [Disclosure]

### [Technical Problem]

The present disclosure is directed to providing a method for preparing high-purity apixaban at high yield, which allows mass production on an industrial scale via an economical, effective and simple process.

### [Technical Solution]

The present disclosure provides a method for preparing apixaban, which includes: 1) a step of reacting a phosphine compound with a compound of Chemical Formula 2 in the presence of an organic solvent; 2) a step of preparing a compound of Chemical Formula 4 by reacting the product with a compound of Chemical Formula 3; and 3) a step of amidating the compound of Chemical Formula 4.

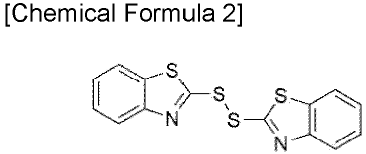

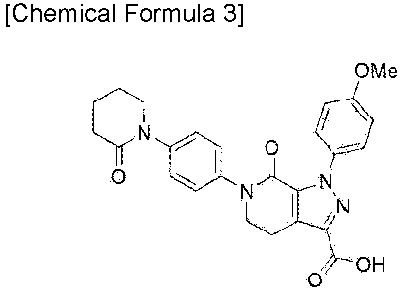

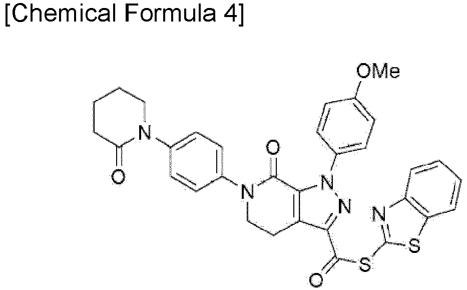

According to an exemplary embodiment of the present disclosure, the phosphine compound may be one selected from a group consisting of a trialkylphosphine, triallylphosphine, a trialkyl phosphite, triallyl phosphite and triphenylphosphine.

According to an exemplary embodiment of the present disclosure, the phosphine compound may be added in an amount of 2-4 equivalents based on the compound of Chemical Formula 2.

According to an exemplary embodiment of the present disclosure, the organic solvent in the step 1) may be one or more selected from a group consisting of dichloromethane, chloroform, toluene, acetone, ethyl acetate, methanol, ethanol, isopropyl alcohol, isopropyl acetate, tetrahydrofuran, N,N-dimethylformamide, N,N-dimethylacetamide, acetonitrile, methyl ethyl ketone, methyl isobutyl ketone and dimethyl sulfoxide.

According to an exemplary embodiment of the present disclosure, the reaction of the step 1) may be performed at -20 to 80 °C for 0.5-5 hours.

According to an exemplary embodiment of the present disclosure, the reaction of the step 2) may be performed at -20 to 80 °C for 0.5-5 hours.

According to an exemplary embodiment of the present disclosure, the amidation of the step 3) may be performed by adding 5-30 equivalents (eq) of anhydrous ammonia, ammonia water or ammonium salt based on the compound of Chemical Formula 4.

According to an exemplary embodiment of the present disclosure, the amidation of the step 3) may be performed at -20 to 80 °C for 0.5-5 hours.

According to an exemplary embodiment of the present disclosure, the compound of Chemical Formula 3 may be obtained by reacting a compound of Chemical Formula 6 with sodium hydroxide in the presence of an organic solvent.

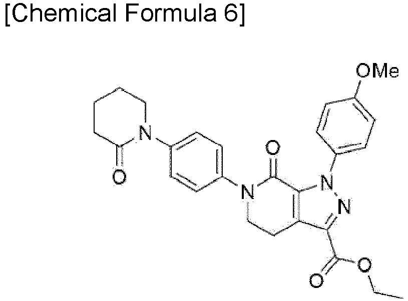

### [Advantageous Effects]

The method of the present disclosure is economical because expensive reagents or solvents are not used. In addition, the method is applicable to mass production on an industrial scale because it requires no special conditions or complicated processes and no hazardous reagents or solvents are used. Furthermore, because no toxic solvent is used in the final step of apixaban synthesis and in a purification process, high-purity apixaban can be prepared stably at high yield without the concern of residual solvents.

### [Brief Description of Drawings]

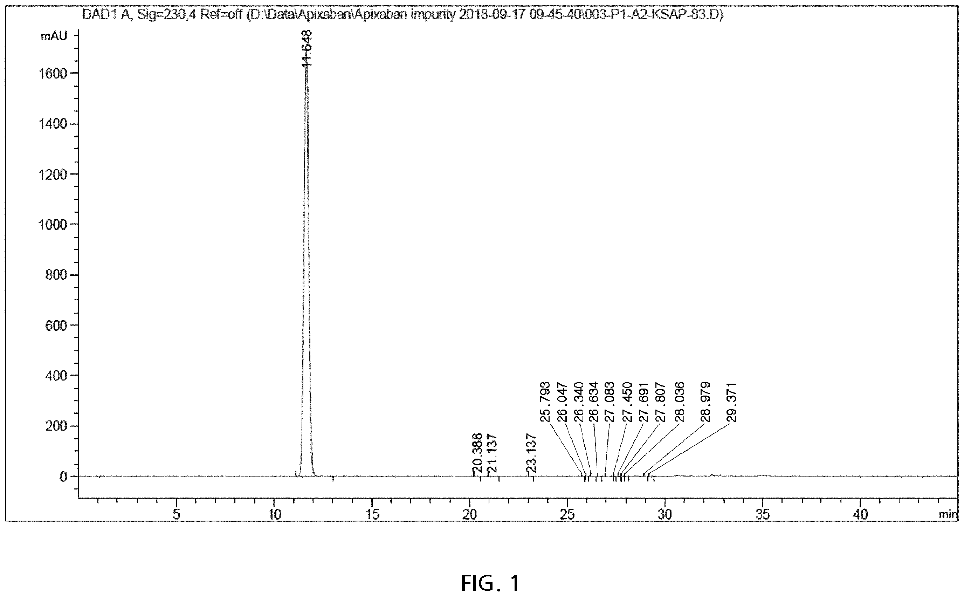

FIG. 1 shows the HPLC analysis result of apixaban prepared by a method of the present disclosure.

### [Best Mode]

Hereinafter, the present disclosure is described in detail.

The present disclosure provides a method for preparing apixaban, which includes: 1) a step of reacting a phosphine compound with a compound of Chemical Formula 2 in the presence of an organic solvent; 2) a step of preparing a compound of Chemical Formula 4 by reacting the product with a compound of Chemical Formula 3; and 3) a step of amidating the compound of Chemical Formula 4.

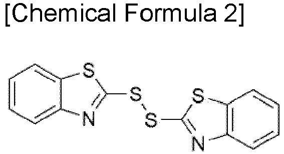

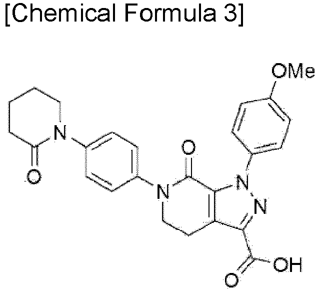

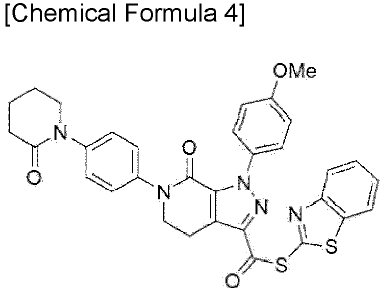

First, the step 1) is a step wherein a phosphine compound is reacted with a compound of Chemical Formula 2 in the presence of an organic solvent.

The phosphine compound may be one selected from a group consisting of a trialkylphosphine, triallylphosphine, a trialkyl phosphite, triallyl phosphite and triphenylphosphine. Specifically, it may be triphenylphosphine.

2-4 equivalents of the phosphine compound may be added based on the compound of Chemical Formula 2. The compound of Chemical Formula 2 may be 2,2'-dithiobis(benzothiazole).

The organic solvent may be one or more selected from a group consisting of dichloromethane, chloroform, toluene, acetone, ethyl acetate, methanol, ethanol, isopropyl alcohol, isopropyl acetate, tetrahydrofuran, N,N-dimethylformamide, N,N-dimethylacetamide, acetonitrile, methyl ethyl ketone, methyl isobutyl ketone and dimethyl sulfoxide.

The reaction may be performed at -20 to 80 °C for 0.5-5 hours.

When the phosphine compound and the compound of Chemical Formula 2 are reacted in the presence of an organic solvent as described above, a benzothiazole phosphine salt represented by Chemical Formula 5 may be produced.

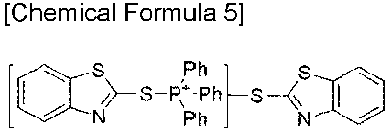

The step 2) is a step wherein a compound of Chemical Formula 4 is prepared by adding a compound of Chemical Formula 3 to the product of the step 1) and conducting reaction.

The reaction may be performed at -20 to 80 °C, specifically at 15-40 °C, for 0.5-5 hours, specifically for 1-1.5 hours.

The compound of Chemical Formula 3 may be 1-(4-methoxyphenyl)-7-oxo-6-(4-2-oxopiperidin-1-yl)phenyl)-4,5,6,7-tetrahydro-1 H-pyrazolo[3,4-c]pyridine-3-carboxylic acid. The compound of Chemical Formula 4 may be 2-benzothiazolyl-1-(4-methoxyphenyl)-7-oxo-6-(4-2-oxopiperidin-1-yl)phenyl)-4, 5,6,7-tetrahydro-1H-pyrazolo[3,4-c]pyridine-3-carboxylate.

Meanwhile, the compound of Chemical Formula 3 may be obtained by reacting the compound of Chemical Formula 6 with sodium hydroxide in the presence of an organic solvent.

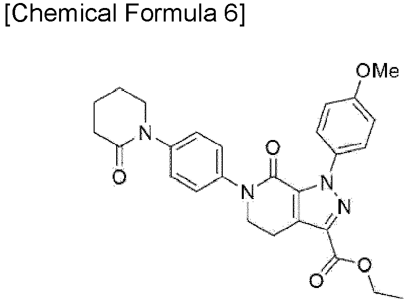

The compound of Chemical Formula 6 may be ethyl-1-(4-methoxyphenyl)-7-oxo-6-(4-2-oxopiperidin-1-yl)phenyl-4,5,6,7-tetrahy dro-1H-pyrazolo[3,4-c]pyridine-3-carboxylate.

The the step 3) is a step wherein the compound of Chemical Formula 4 prepared in the step 2) is amidated.

The amidation of the compound of Chemical Formula 4 may be performed in the presence of 5-30 equivalents (eq), specifically 5-15 equivalents, of anhydrous ammonia, ammonia water or ammonium salt.

In addition, the amidation may be performed at -20 to 80 °C, specifically at 15-40 °C, for 0.5-5 hours, specifically for 1-1.5 hours.

Preferred preparation processes of apixaban according to the present disclosure are shown in Schemes 6 and 7.

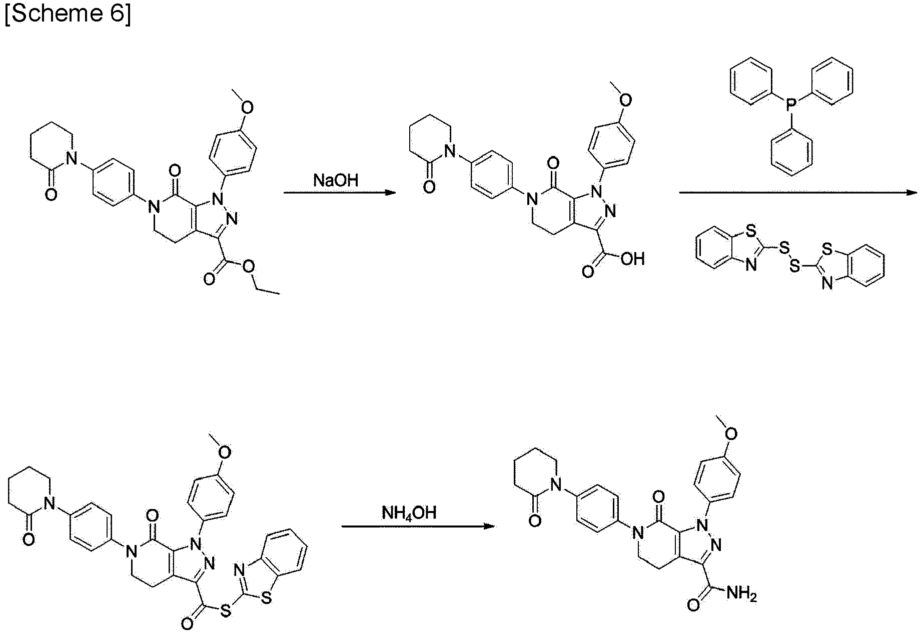

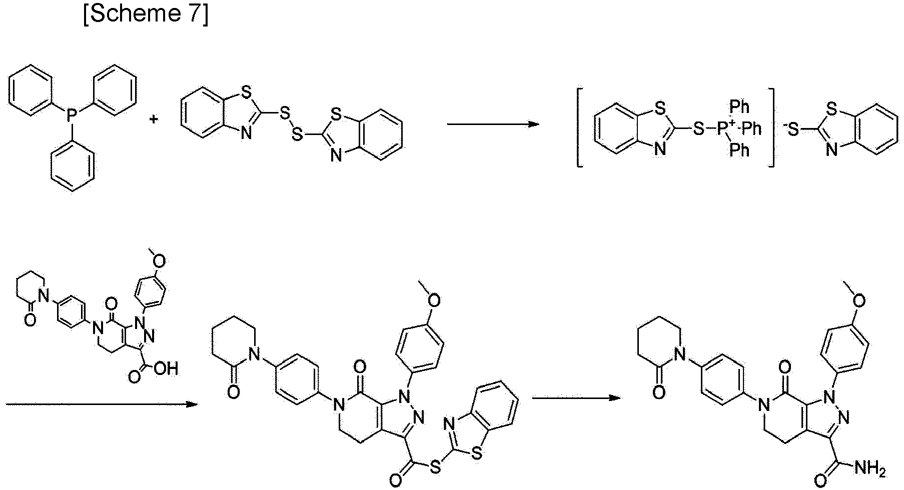

The starting material (Chemical Formula 6) used in Scheme 6 is a compound known as an important intermediate of apixaban and is easily commercially available.

The product prepared according to the method described above can be crystallized easily using a mixture organic solvent after a workup process followed by removal of the reaction solvent under reduced pressure. Specifically, the mixture organic solvent may be a mixture solvent of ethyl acetate and chloroform.

Unlike the existing technologies, the preparation method according to the present disclosure does not include a process requiring an expensive metal catalyst, a process requiring special production facilities for high-pressure reactions, or a dangerous process that may affect the safety of operators. In addition, according to the method of the present disclosure, apixaban may be obtained with high yield of 90% or higher and high purity of 99.9% or higher at room temperature in short time.

Hereinafter, the method for preparing apixaban according to the present disclosure will be described in more detail through examples and a comparative example.

### <Examples>

### Example 1. Preparation of 1-(4-methoxyphenyl)-7-oxo-6-(4-2-oxopiperidin-1-yl)phenyl)-4,5,6,7-tetrahydro-1 H-pyrazolo[3,4-c]pyridine-3-carboxylic acid (Chemical Formula 3)

135 g of ethyl-1-(4-methoxyphenyl)-7-oxo-6-(4-2-oxopiperidin-1-yl)phenyl-4,5,6,7-tetrahy dro-1H-pyrazolo[3,4-c]pyridine-3-carboxylate (Chemical Formula 6) was suspended in 675 mL of dichloromethane and 675 mL of methanol. After adding 22.11 g of sodium hydroxide, the reaction mixture was stirred at 60 ± 5 °C for 2 hours. After cooling the reaction mixture to 0 °C, pH was adjusted to 2-3 by adding 120 mL of 1 N HCI dropwise. After concentrating the reaction mixture and adding 1 L of purified water, 115 g (yield: 90%) of a target compound was obtained by stirring at room temperature for 2 hours and filtering the mixture.

### Example 2. Preparation of 2-benzothiazolyl-1-(4-methoxyphenyl)-7-oxo-6-(4-2-oxopiperidin-1-yl)phenyl)-4, 5,6,7-tetrahydro-1H-pyrazolo[3,4-c]pyridine-3-carboxylate (Chemical Formula 4)

86 g of triphenylphosphine and 108 g of 2,2'-dithiobis(benzothiazole) (Chemical Formula 2) were suspended in 500 mL of dichloromethane and stirred at room temperature for 1.5 hours. After adding 50 g of 1-(4-methoxyphenyl)-7-oxo-6-(4-2-oxopiperidin-1-yl)phenyl)-4,5,6,7-tetrahydro-1 H-pyrazolo[3,4-c]pyridine-3-carboxylic acid (Chemical Formula 3), the reaction mixture was stirred at room temperature for 1 hour. After the reaction was completed, an MC layer was extracted by adding 1.4 L of water. After drying with anhydrous magnesium sulfate and filtering, the filtrate was concentrated under reduced pressure. 62 g (yield: 94%) of a target compound was obtained by recrystallizing the concentrated residue by adding 100 mL of acetone and 540 mL of isopropyl alcohol.

¹H NMR (300 MHz, CDCl₃): δ 8.10 (d, 1H), 7.95 (d, 1H), 7.56 (m, 4H), 7.35 (dd, 4H), 6.98 (d, 2H), 4.15 (t, 2H), 3.83 (s, 3H), 3.60 (t, 2H), 3.34 (t, 2H), 2.55 (t, 2H), 1.93 (m, 4H).

### Example 3. Preparation of apixaban

1.4 kg of 2-benzothiazolyl-1-(4-methoxyphenyl)-7-oxo-6-(4-2-oxopiperidin-1-yl)phenyl)-4, 5,6,7-tetrahydro-1H-pyrazolo[3,4-c]pyridine-3-carboxylate (Chemical Formula 4) was suspended in 10 L of dichloromethane. After adding 309 mL of ammonia water, the reaction mixture was stirred at room temperature for 1 hour. After the reaction was completed, an organic layer was extracted by adding 10 L of purified water to the reaction mixture. After adding 300 mL of 3 N HCI to the extracted organic layer, an aqueous layer was separated. The separated aqueous layer was adjusted to pH 7.5 ± 0.5 by adding 100 mL of 2 N NaOH. The extracted organic layer was dried with anhydrous magnesium sulfate and then filtered. The filtrate was concentrated under reduced pressure. 0.9 kg (yield: 90%) of a target compound was obtained by recrystallizing the concentrated residue by adding 1.7 L of ethyl acetate and 4.8 L of chloroform.

A result of analyzing the obtained synthesis product by HPLC is shown in FIG. 1. The HPLC analysis was conducted using an XBridge BEH C18 column at a flow rate of 1.0 mL/min. A UV absorption spectrophotometer (measurement wavelength: 230 nm) was used as a UV detector. A phosphate buffer (pH 4.0) was used as mobile phase A, and acetonitrile was used as mobile phase B. 50% acetonitrile was used as a diluent.

¹H NMR (300 MHz, CDCl₃): δ 7.49 (d, 2H), 7.35 (d, 2H), 7.26 (d, 2H), 6.95 (d, 2H), 6.85 (s, 1H), 5.63 (s, 1H), 4.13 (t, 2H), 3.81 (s, 3H), 3.59 (t, 3H), 3.39 (t, 2H), 2.55 (t, 2H), 1.93 (m, 4H).

HPLC: 99.95%.

### <Comparative Example>

### Comparative Example 1. Preparation of apixaban (using sealed tube)

In a sealed tube, 10 g of ethyl-1-(4-methoxyphenyl)-7-oxo-6-(4-2-oxopiperidin-1-yl)phenyl-4,5,6,7-tetrahy dro-1H-pyrazolo[3,4-c]pyridine-3-carboxylate (Chemical Formula 6) was suspended in 80 mL of a 5% ammonia ethylene glycol solution and stirred at 120 °C for 4 hours. 6.3 g (yield: 63%) of a target compound was obtained by conducting column chromatography with Mc : MeOH = 10 : 1.

HPLC: 98% or higher.

Although the specific exemplary embodiments of the present disclosure have been described above, various changes or modifications can be made without departing from the scope of the present disclosure. Such changes or modifications belong to the scope of the present disclosure defined by the appended claims.

## Claims

1. A method for preparing apixaban, comprising:
1) a step of reacting a phosphine compound with a compound of Chemical Formula 2 in the presence of an organic solvent;
2) a step of preparing a compound of Chemical Formula 4 by reacting the product with a compound of Chemical Formula 3; and
3) a step of amidating the compound of Chemical Formula 4.

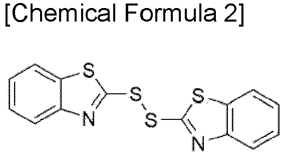

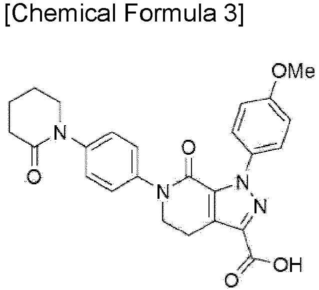

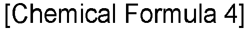

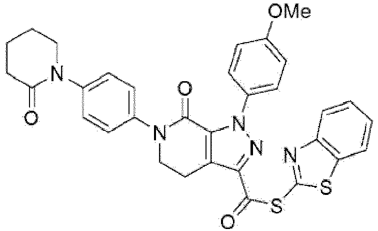

2. The method for preparing apixaban according to claim 1, wherein the phosphine compound is one selected from a group consisting of a trialkylphosphine, triallylphosphine, a trialkyl phosphite, triallyl phosphite and triphenylphosphine.

3. The method for preparing apixaban according to claim 1, wherein the phosphine compound is added in an amount of 2-4 equivalents based on the compound of Chemical Formula 2.

4. The method for preparing apixaban according to claim 1, wherein the organic solvent in the step 1) is one or more selected from a group consisting of dichloromethane, chloroform, toluene, acetone, ethyl acetate, methanol, ethanol, isopropyl alcohol, isopropyl acetate, tetrahydrofuran, N,N-dimethylformamide, N,N-dimethylacetamide, acetonitrile, methyl ethyl ketone, methyl isobutyl ketone and dimethyl sulfoxide.

5. The method for preparing apixaban according to claim 1, wherein the reaction of the step 1) is performed at -20 to 80 °C for 0.5-5 hours.

6. The method for preparing apixaban according to claim 1, wherein the reaction of the step 2) is performed at -20 to 80 °C for 0.5-5 hours.

7. The method for preparing apixaban according to claim 1, wherein the amidation of the step 3) is performed by adding 5-30 equivalents (eq) of anhydrous ammonia, ammonia water or ammonium salt based on the compound of Chemical Formula 4.

8. The method for preparing apixaban according to claim 1, wherein the amidation of the step 3) is performed at -20 to 80 °C for 0.5-5 hours.

9. The method for preparing apixaban according to claim 1, wherein the compound of Chemical Formula 3 is obtained by reacting a compound of Chemical Formula 6 with sodium hydroxide in the presence of an organic solvent.
